(19) Europäisches Patentamt
European Patent Office
Office européen des brevets

(11) Veröffentlichungsnummer: **0 005 204**
**A2**

# EUROPÄISCHE PATENTANMELDUNG

(21) Anmeldenummer: **79101154.7**

(22) Anmeldetag: **17.04.79**

(51) Int. Cl.$^2$: **A 01 N 5/00,** C 07 D 277/00

(30) Priorität: **27.04.78 DE 2818504**

(43) Veröffentlichungstag der Anmeldung: **14.11.79**
**Patentblatt 79/23**

(84) Benannte Vertragsstaaten: **BE CH DE FR GB IT NL**

(71) Anmelder: **BAYER Aktiengesellschaft, Zentralbereich Patente,Marken und Lizenzen Bayerwerk, D-5090 Leverkusen 1 (DE)**

(72) Erfinder: **Grohe, Klaus, Dr., Am Wasserturm 10, D-5068 Odenthal (DE)**
Erfinder: **Lürssen, Klaus, Dr., August-Kierspel-Strasse 89, D-5060 Bergisch-Gladbach 2 (DE)**

(54) **2-Thiazolon-5-carbonsäure-Derivate enthaltende Mittel zur Regulierung des Pflanzenwachstums, Verfahren zu deren Herstellung sowie deren Verwendung.**

(57) Die teilweise bekannten 2-Thiazolon-5-carbonsäure-Derivate der Formel

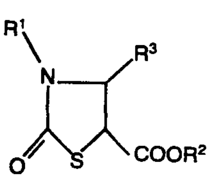

(I)

in welcher

$R^1$ für Wasserstoff, Alkyl, Alkoxyalkyl, Alkylmercaptoalkyl, durch heterocyclische Reste substituiertes Alkyl, durch Alkylcarbonyl substituiertes Alkyl, durch Arylcarbonyl, welches seinerseits im Arylteil durch Halogen, Alkyl, Halogenalkyl, Alkoxy, Alkylthio und/oder Nitro substituiert sein kann, ferner für Alkenyl, Cycloalkyl, gegebenenfalls substituiertes Aryl, gegebenenfalls im Arylteil substituiertes Aralkyl oder für die Gruppierung

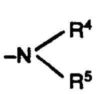 steht,

in welcher

$R^4$ für Wasserstoff, Alkyl oder Aryl steht,

$R^5$ für Alkyl, Alkylcarbonyl, Carbalkoxy, Alkylsulfonyl oder Arylsulfonyl steht und außerdem

$R^4$ und $R^5$ gemeinsam mit dem angrenzenden Stickstoffatom für einen 5- bis 7-gliedrigen heterocyclischen Ring stehen,

$R^2$ für Wasserstoff, gegebenenfalls durch Cycloalkyl, Alkoxy, Alkylmercapto, Phenoxyl, Arylthio oder Cyano substituiertes Alkyl, gegebenenfalls durch Alkyl substituiertes Cycloalkyl oder für gegebenenfalls substituiertes Aralkyl steht und

$R^3$ für Wasserstoff, Alkyl, Alkoxyalkyl, Halogenalkyl, Cycloalkyl, gegebenenfalls substituiertes Aryl oder gegebenenfalls substituiertes Aralkyl steht,

besitzen starke pflanzenwachstumsregulierende Eigenschaften.

EP 0 005 204 A2

00005204



BEZEICHNUNG GEÄNDERT
siehe Titelseite

BAYER AKTIENGESELLSCHAFT
Zentralbereich
Patente, Marken und Lizenzen

5090 Leverkusen, Bayerwerk
Dü/Kü
IIb

Mittel zur Regulierung des Pflanzenwachstums

Die vorliegende Erfindung betrifft die Verwendung von teilweise bekannten 2-Thiazolon-5-carbonsäure-Derivaten als Wirkstoffe zur Regulierung des Pflanzenwachstums.

Zahlreiche 2-Thiazolon-5-carbonsäureester und deren Verwendung als Fungizide sind bereits bekannt (vgl. DT-OS 22 53 027).

Es ist weiterhin bekannt, daß Bernsteinsäure-2,2-dimethylhydrazid pflanzenwachstumsregulierende Eigenschaften besitzt (vgl. R. Wegler "Chemie der Pflanzenschutz- und Schädlingsbekämpfungsmittel", Band 2, Seite 265). Die Wirkung dieses Stoffes ist jedoch, vor allem bei niedrigen Aufwandmengen, nicht immer befriedigend.

Außerdem ist bekannt geworden, daß bestimmte 2-Halogenäthyl-trialkylammonium-halogenide pflanzenwuchsregulierende Eigenschaften aufweisen (vgl. US-Patentschrift 3 156 554). So läßt sich z.B. mit Hilfe von (2-Chloräthyl)-trimethyl-ammonium-chlorid eine Beeinflussung des Pflanzenwachstums erzielen. Allerdings ist auch die Wirksamkeit dieses Stoffes, vor allem bei niedrigen Aufwandmengen, nicht immer ausreichend.

Es wurde nun gefunden, daß die teilweise bekannten 2-Thiazolon-5-carbonsäure-Derivate der Formel

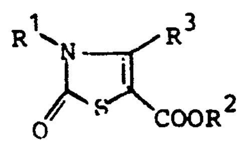

(I)

in welcher

$R^1$ für Wasserstoff, Alkyl, Alkoxyalkyl, Alkylmercaptoalkyl, durch heterocyclische Reste substituiertes Alkyl, durch Alkylcarbonyl substituiertes Alkyl, durch Arylcarbonyl, welches seinerseits im Arylteil durch Halogen, Alkyl, Halogenalkyl, Alkoxy, Alkylthio und/oder Nitro substituiert sein kann, substituiertes Alkyl, ferner für Alkenyl, Cycloalkyl, gegebenenfalls substituiertes Aryl, gegebenenfalls im Arylteil substituiertes Aralkyl oder für die Gruppierung

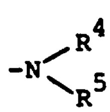 steht,

in welcher

$R^4$ für Wasserstoff, Alkyl oder Aryl steht,

$R^5$ für Alkyl, Alkylcarbonyl, Carbalkoxy, Alkylsulfonyl oder Arylsulfonyl steht und außerdem

$R^4$ und $R^5$ gemeinsam mit dem angrenzenden Stickstoffatom für einen 5- bis 7-gliedrigen heterocyclischen Ring stehen,

$R^2$ für Wasserstoff, gegebenenfalls durch Cycloalkyl, Alkoxy, Alkylmercapto, Phenoxyl, Arylthio oder Cyano substituiertes Alkyl, gegebenenfalls durch Alkyl substituiertes Cycloalkyl oder für gegebenenfalls substituiertes Aralkyl steht und

$R^3$ für Wasserstoff, Alkyl, Alkoxyalkyl, Halogenalkyl, Cycloalkyl, gegebenenfalls substituiertes Aryl oder gegebenenfalls substituiertes Aralkyl steht,

starke pflanzenwachstumsregulierende Eigenschaften besitzen.

Überraschenderweise zeigen die erfindungsgemäß verwendbaren 2-Thiazolon-5-carbonsäure-Derivate der Formel (I) eine bessere pflanzenwachstumsregulierende Wirksamkeit als das aus dem Stand der Technik bekannte Bernsteinsäure -2,2-dimethylhydrazid und das ebenfalls bekannte (2-Chloräthyl)-trimethylammonium-chlorid, welche anerkannt gut wirksame Stoffe gleicher Wirkungsart sind. Die erfindungsgemäß verwendbaren Stoffe stellen somit eine wertvolle Bereicherung der Technik dar.

Die erfindungsgemäß zu verwendenden 2-Thiazolon-5-carbonsäure-Derivate sind durch die Formel (I) allgemein definiert. In dieser Formel steht $R^1$ vorzugsweise für Wasserstoff, geradkettiges oder verzweigtes Alkyl mit 1 bis 4 Kohlenstoffatomen, geradkettiges oder verzweigtes Alkoxyalkyl mit 1 bis 4 Kohlenstoffatomen in der Alkylgruppe und 1 bis 4 Kohlenstoffatomen im Alkoxyteil, geradkettiges oder verzweigtes Alkylmercaptoalkyl mit 1 bis 4 Kohlenstoffatomen in der Alkylgruppe und 1 bis 4 Kohlenstoffatomen im Alkylmercapto- Teil, für ein oder mehrfach durch gesättigte oder ungesättigte heterocyclische Reste mit 5 bis 7 Ringgliedern substituiertes geradkettiges oder verzweigtes Alkyl mit 1 bis 4 Kohlenstoffatomen, wobei als heterocyclische Reste vorzugsweise solche in Frage kommen, die 1 bis 3 Heteroatome, wie Stickstoff, Sauerstoff und/oder Schwefel enthalten. Als Beispiele für derartige heterocyclische Reste seien genannt: Pyridinyl, Piperidinyl, Pyrrolidinyl, Piperazinyl, Pyrazolidinyl, Morpholinyl, Thiamorpholinyl, Thiazclyl, Imidazolyl, Pyrazolyl, 1,2,4-Triazolyl und 1,2,3-Triazolyl. Ferner steht $R^1$ vorzugsweise für durch Alkylcarbonyl mit bis zu 5 Kohlenstoffatomen substituiertes geradkettiges oder verzweigtes Alkyl mit 1 bis 4 Kohlenstoffatomen. Weiterhin steht $R^1$ vorzugsweise für durch Phenylcarbonyl oder Naphthylcarbonyl substituiertes geradkettiges oder verzweigtes Alkyl mit 1 bis 4 Kohlenstoffatomen, wobei der Phenyl- bzw. Naphthylrest jeweils noch seinerseits substituiert sein kann durch Halogen, Alkyl mit 1 bis 4 Kohlenstoffatomen, Halogenalkyl mit 1 bis 4 Kohlenstoffatomen und 1 bis 5 Halogenatomen, insbesondere Fluor oder Chloratomen , Alkoxy mit 1 bis 4 Kohlenstoffatomen, Alkylthio mit 1 bis 4 Kohlenstoffatomen und/oder Nitro. Außerdem steht $R^1$ vorzugsweise für geradkettiges oder verzweigtes Alkenyl mit 2 bis 4 Kohlenstoffatomen, Cycloalkyl mit 5 bis 7 Kohlenstoffatomen, gegebenenfalls durch Halogen, Alkyl mit 1 bis 4 Kohlenstoffatomen, Halogenalkyl mit 1 bis 4 Kohlenstoffatomen und 1 bis 5 Halogenatomen, insbesondere Fluor- oder Chloratomen , Alkoxy mit 1 bis 4 Kohlenstoffatomen, Alkylthio mit 1 bis 4 Kohlenstoffatomen, und/oder Nitro substituiertes Aryl mit 6 oder 10 Kohlenstoffatomen, wobei Phenyl beispielhaft genannt sei. Ferner steht $R^1$ vorzugsweise für

gegebenenfalls im Arylteil durch Halogen, Alkyl mit 1 bis 4 Kohlenstoffatomen, Halogenalkyl mit 1 bis 4 Kohlenstoffatomen und 1 bis 5 Halogenatomen, insbesondere Fluor - oder Chloratomen , Alkoxy mit 1 bis 4 Kohlenstoffatomen, Alkylthio mit 1 bis 4 Kohlenstoffatomen und/oder Nitro substituiertes Aralkyl mit 6 oder 10 Kohlenstoffatomen im Arylteil und 1 bis 4 Kohlenstoffatomen im Alkylteil, wobei Benzyl beispielhaft genannt sei. Darüberhinaus steht $R^1$ für die Gruppierung $-N(R^5)-R^4$. Hierin steht $R^4$ vorzugsweise für Wasserstoff, geradkettiges oder verzweigtes Alkyl mit 1 bis 6 Kohlenstoffatomen und für Aryl mit 6 oder 10 Kohlenstoffatomen, wobei Phenyl beispielhaft genannt sei. $R^5$ steht vorzugsweise für geradkettiges oder verzweigtes Alkyl mit 1 bis 6 Kohlenstoffatomen, Alkylcarbonyl mit bis zu 5 Kohlenstoffatomen, Carbalkoxy mit bis zu 5 Kohlenstoffatomen, Alkylsulfonyl mit 1 bis 4 Kohlenstoffatomen und für Arylsulfonyl mit 6 oder 10 Kohlenstoffatomen. Außerdem stehen $R^4$ und $R^5$ gemeinsam mit dem angrenzenden Stickstoffatom, vorzugsweise für einen gesättigten oder ungesättigten heterocyclischen Ring mit 5 bis 7 Ringgliedern, wobei 1 oder 2 Kohlenstoffatome im Ring ersetzt sein können durch Sauerstoff, Schwefel oder $-SO_2-$. Als Beispiele für derartige über Stickstoff gebundene heterocyclische Reste seien genannt: Piperidinyl, Pyrrolidinyl, Morpholinyl, Thiomorpholinyl und Hexahydroazepinyl. - In der Formel (I) steht $R^2$ vorzugsweise für Wasserstoff, gegebenenfalls durch Cycloalkyl mit 5 bis 7 Kohlenstoffatomen, Alkoxy oder Alkylmercapto mit 1 bis 4 Kohlenstoffatomen, Phenoxy, Phenylthio oder Cyano substituiertes Alkyl mit 1 bis 16 Kohlenstoffatomen, gegebenenfalls durch Alkyl mit 1 bis 4 Kohlenstoffatomen substituiertes Cycloalkyl mit 5 bis 7 Kohlenstoffatomen und für gebebenenfalls im Arylteil durch Halogen, Alkyl,

mit 1 bis 4 Kohlenstoffatomen, Halogenalkyl mit 1 bis 4 Kohlenstoffatomen und 1 bis 5 Halogenatomen, insbesondere Fluor- oder Chloratomen , Alkoxy mit 1 bis 4 Kohlenstoffatomen, Alkylthio mit 1 bis 4 Kohlenstoffatomen und/oder Nitro substituiertes Aralkyl mit 6 oder 10 Kohlenstoffatomen im Arylteil und 1 bis 4 Kohlenstoffatomen im Alkylteil. $R^3$ steht vorzugsweise für Wasserstoff, geradkettiges oder verzweigtes Alkyl mit 1 bis 4 Kohlenstoffatomen, geradkettiges oder verzweigtes Alkoxyalkyl mit 1 bis 4 Kohlenstoffatomen im Alkylteil und 1 bis 4 Kohlenstoffatomen im Alkoxyteil, geradkettiges oder verzweigtes Halogenalkyl mit 1 bis 4 Kohlenstoffatomen und 1 bis 5 Halogenatomen, insbesondere Fluor-oder Chloratomen, ferner für Cycloalkyl mit 5 bis 7 Kohlenstoffatomen, gegebenenfalls durch Halogen, Alkyl mit 1 bis 4 Kohlenstoffatomen, Halogenalkyl mit 1 bis 4 Kohlenstoffatomen und 1 bis 5 Halogenatomen, insbesondere Fluor- oder Chloratomen , Alkoxy mit 1 bis 4 Kohlenstoffatomen, Alkylthio mit 1 bis 4 Kohlenstoffatomen und/oder Nitro substituiertes Aryl mit 6 oder 10 Kohlenstoffatomen, wobei Phenyl beispielhaft genannt sei. Ferner steht $R^3$ vorzugsweise für gegebenenfalls im Arylteil durch Halogen, Alkyl mit 1 bis 4 Kohlenstoffatomen, Halogenalkyl mit 1 bis 4 Kohlenstoffatomen und 1 bis 5 Halogenatomen, insbesondere Fluor- oder Chloratomen , Alkoxy mit 1 bis 4 Kohlenstoffatomen, Alkylthio mit 1 bis 4 Kohlenstoffatomen und/oder Nitro substituiertes Aralkyl mit 6 oder 10 Kohlenstoffatomen im Arylteil und 1 bis 4 Kohlentoffatomen im Alkylteil, wobei Benzyl beispielhaft genannt sei.

Besonders bevorzugt verwendbar sind solche Verbindungen der Formel (I), in denen $R^1$ für Wasserstoff, Methyl, Äthyl,

n-Propyl, iso-Propyl, n-Butyl, sec.-Butyl, tert.-Butyl, Methoxymethyl,Methoxyäthyl durch Pyridinyl, Piperdinyl, Pyrrolidinyl, Piperazinyl, Pyrazolidinyl, Morpholinyl, Thiamorpholinyl, Thiazolyl, Imidazolyl, 1,2,4-Triazolyl oder 1,2,3-Triazolyl substituiertes Methyl oder Äthyl steht, ferner für durch Methylcarbonyl oder Äthylcarbonyl substituiertes Methyl oder Äthyl steht, weiterhin für durch Phenylcarbonyl, 4-Chlorphenylcarbonyl, 4-Methylphenylcarbonyl- 2-Methylphenylcarbonyl,4-Trifluormethylphenylcarbonyl, 4-Trichlormethylphenylcarbonyl, 4-Methoxyphenylcarbonyl, 3-Methoxyphenylcarbonyl, 4-Methylthienylcarbonyl oder 3-Nitrophenylcarbonyl substituiertes Methyl oder Äthyl steht, außerdem für Allyl, Cyclopentyl, Cyclohexyl, Phenyl,Naphthyl, 2-Chlorphenyl, 3-Chlorphenyl, 4-Chlorphenyl, 4-Methylphenyl, 4-Trifluormethylphenyl, 4-Trichlormethylphenyl, 4-Methoxyphenyl, 4-Methylthiophenyl, 3-Nitrophenyl, Benzyl, 4-Chlorbenzyl, 3-Chlorbenzyl, 2-Chlorbenzyl, 4-Methylbenzyl, 4-Trifluormethylbenzyl, 4-Trichlormethylbenzyl, 4-Methoxybenzyl, 4-Methylthiobenzyl oder 3-Nitrobenzyl steht, darüberhinaus für Methylamino, Äthylamino, n-Propylamino, n-Butylamino, Phenylamino, Dimethylamino, Diäthylamino, Di-n-propylamino, Phenylmethyl-amino, Piperidinyl, Pyrrolidinyl, Morpholinyl, Thiomorpholinyl oder Hexahydroazepinyl steht, $R^2$ für Wasserstoff, Methyl, Äthyl, n-Propyl, iso-Propyl, n-Butyl, iso-Butyl, sec.-Butyl, tert.Butyl, n-Pentyl, 2-Äthyl-butyl, 2-Methylbutyl- n-Hexyl, n-Heptyl, n-Octyl, n-Dodecyl, durch Methoxy ,Äthoxy, Isopropoxy, Phenoxy, Cyano, Cyclohexyl oder Cyclopentyl substituiertes Methyl oder Äthyl, Cyclopentyl, Cyclohexyl, Methylcyclohexyl, 3,3,5-Trimethylcyclohexyl, Benzyl, 4-Chlorbenzyl, 2-Chlorbenzyl, 4-Methylbenzyl, 4-Trifluormethylbenzyl, 4-Trichlormethylbenzyl, 4-Methoxybenzyl, 4-Methylthiobenzyl, 3-Nitrobenzyl oder

ß-(2,6-Dichlorphenyläthyl) steht und $R^3$ für Wasserstoff, Methyl, Äthyl, n-Propyl, iso-Proypl, n-Butyl, sek-Butyl, tert.-Butyl, durch Methoxy, Äthoxy, Isopropoxy, Propoxy oder n-Butoxy substituiertes Methyl, Äthyl, Propyl oder Butyl, Chlormethyl, Dichlormethyl, Trichlormethyl, Cyclohexyl, Cyclopentyl, Phenyl, Naphthyl, 4-Chlorphenyl- 3-Chlorphenyl, 2-Chlorphenyl, 4-Methylphenyl, 4-Trifluormethylphenyl, 4-Methylthiophenyl, 3-Nitrophenyl, 4-Chlorbenzyl, 4-Methylbenzyl, 4-Trifluormethylbenzyl, 4-Methoxybenzyl, 4-Methylthiobenzyl oder 3 Nitrobenzyl steht.

Als Beispiele für Verbindungen der Formel (I) seinen im einzelnen genannt:

3-Methyl-4-methyl-2-thiazolon-5-carbonsäureäthylester
3-Benzyl-4-methyl-2-thiazolon-5-carbonsäureäthylester
4-Methyl-2-thiazolon-5-carbonsäureäthylester
4-Methyl-2-thiazolon-5-carbonsäure-isopropylester
4-Methyl-2-thiazolon-5-carbonsäure-(ß-phenyl)-äthylester
4-Methyl-3-phenyl-2-thiazolon-5-carbonsäureäthylester
4-Methyl-3-phenyl-2-thiazolon-5-carbonsäuremethylester
4-Methyl-2-thiazolon-5-carbonsäure-n-dodecylester
3-(2,6-Dichlorphenyl)-4-methyl-2-thiazolon-5-carbonsäureäthylester
4-Äthyl-3-phenyl-2-thiazolon-5-carbonsäureäthylester
4-Methyl-2-thiazolon-5-carbonsäure-benzylester
4-Methyl-2-thiazolon-5-carbonsäure-n-butylester
4-Methyl-2-thiazolon-5-carbonsäure-cyclohexylester

4-Methyl-2-thiazolon-5-carbonsäure-methylester
4-Trichlormethyl-2-thiazolon-5-carbonsäuremethylester
4-Methyl-2-thiazolon-5-carbonsäure-cyclohexylmethylester
4-Methyl-2-thiazolon-5-carbonsäure-(ß-äthoxy)-äthylester
4-Methyl-2-thiazolon-5-carbonsäure-(ß-isopropoxy)-äthylester
4-Methyl-2-thiazolon-5-carbonsäure-sec.-butylester
4-Methyl-2-thiazolon-5-carbonsäure-n-hexylester
4-Methyl-2-thiazolon-5-carbonsäure-(ß-cyano)-äthylester
4-Methyl-2-thiazolon-5-carbonsäure-n-decylester
4-Methyl-2-thiazolon-5-carbonsäure-(ß-phenoxy)-äthylester
3-Methyl-4-methyl-2-thiazolon-5-carbonsäure-cyclohexylester
3-Phenyl-4-methyl-2-thiazolon-5-carbonsäure-cyclohexylester
3-Cyclohexyl-4-methyl-2-thiazolon-5-carbonsäureäthylester
3-Cyclopentyl-4-methyl-2-thiazolon-5-carbonsäureäthylester
4-Methyl-3-(2-methyl-phenyl)-2-thiazolon-5-carbonsäureäthylester
4-Methyl-3-naphthyl-2-thiazolon-5-carbonsäuremethylester
4-Methyl-2-thiazolon-5-carbonsäure-ß-(2,6-dichlorphenyl)-äthylester
4-Phenyl-2-thiazolon-5-carbonsäuremethylester
4-Naphthyl-2-thiazolon-5-carbonsäureäthylester
3-Methyl-4-(2,6-dichlorphenyl)-2-thiazolon-5-carbonsäureäthylester
3-Methyl-4-naphthyl-2-thiazolon-5-carbonsäureäthylester

3-Methyl-2-thiazolon-5-carbonsäureäthylester
4-Hexyl-3-methyl-2-thiazolon-5-carbonsäureäthylester
3-Hexyl-4-methyl-2-thiazolon-5-carbonsäureäthylester
4-Methyl-2-thiazolon-5-carbonsäure-octadecylester.
3-Methylamino-4-methyl-2-thiazolon-5-carbonsäureäthylester
3-Äthylamino-4-methyl-2-thiazolon-5-carbonsäureäthylester
3-n-Propylamino-4-methyl-2-thiazolon-5-carbonsäureäthylester
3-Isopropylamino-4-methyl-2-thiazolon-5-carbonsäureäthylester
3-Phenylamino-4-methyl-2-thiazolon-5-carbonsäureäthylester
3-Dimethylamino-4-methyl-2-thiazolon-5-carbonsäureäthylester
3-Diäthylamino-4-methyl-2-thiazolon-5-carbonsäureäthylester
3-(Phenyl-methyl-amino)-4-methyl-2-thiazolon-5-carbonsäureäthylester
3-Äthyl-4-methyl-2-thiazolon-5-carbonsäure.

Die erfindungsgemäß verwendbaren 2-Thiazolon-5-carbonsäure-Derivate der Formel (I) sind teilweise bekannt (vgl. Proc. Indian Akad.Sci, 22A, 362-378 (1945), J.Pharm. Soc. Japan 76 , 301-305 (1956), DT-OS 21 37 649 und DT-OS 22 53 027). Einzelne der erfindungsgemäß verwendbaren Wirkstoffe sind neu, sie können jedoch nach prinzipiell bekannten Verfahren in einfacher Weise hergestellt werden.

So erhält man Verbindungen der Formel (I), indem man

a) ß-Aminoacrylsäureester der Formel

$$R^1 - NH - \overset{R^3}{\overset{|}{C}} = CH - COO\,R^6 \qquad (II)$$

in welcher

$R^1$ und $R^3$ die oben angegebene Bedeutung haben und

$R^6$ außer für Wasserstoff für die gleichen Reste wie $R^2$ steht,

mit Chlorcarbonylsulfenylchlorid der Formel

$$Cl - \overset{O}{\overset{\|}{C}} - S - Cl \qquad (III)$$

gegebenenfalls in Gegenwart eines inerten Verdünnungsmittels, wie Chlorbenzol, und gegebenenfalls in Gegenwart eines säurebindenden Mittels, wie Pyridin, bei Temperaturen zwischen $0^oC$ und $200^oC$ umsetzt,

oder indem man

b) 2-Thiazolon-5-carbonsäureester der Formel

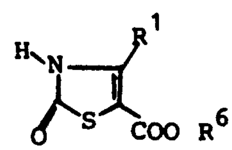

(IV)

in welcher

$R^1$ und $R^6$ die oben angegebene Bedeutung haben,

mit Halogenverbindungen der Formel

$$R^7 - X \qquad (V)$$

in welcher

$R^7$ für Alkyl, Alkenyl, Cycloalkyl oder gegebenenfalls im Arylteil substituiertes Aralkyl steht und

X für Chlor, Brom oder Jod steht,

in Gegenwart eines inerten organischen Lösungsmittels, wie Acetonitril, Dioxan oder Toluol, und in Gegenwart eines säurebindenden Mittels, wie Triäthylamin oder Pyridin, bei Temperaturen zwischen 60°C und 160°C, vorzugsweise zwischen 70°C und 120°C umsetzt,

oder indem man

c) 2-Thiazolon-5-carbonsäureester der Formel

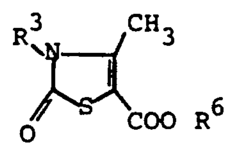

(VI)

in welcher

$R^3$ und $R^6$ die oben angegebene Bedeutung haben,

mit elementarem Chlor in Gegenwart eines Lösungsmittels, wie Chloroform, Tetrachlorkohlenstoff oder Sulfurylchlorid, bei Temperaturen zwischen 20°C und 120°C, vorzugsweise 60° C und 80°C umsetzt.

Diejenigen Verbindungen der Formel (I), in denen $R^2$ für Wasserstoff steht erhält man, indem man Ester der Formel

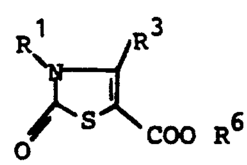

(VII)

in welcher

$R^1$, $R^3$ und $R^6$ die oben angegebene Bedeutung haben,

mit Hilfe von Basen, wie Natriumhydroxid oder Kaliumhydroxid, in Gegenwart eines Lösungsmittels, wie eines Alkohol/Wasser-Gemisches, bei Temperaturen zwischen 0°C und 100°C verseift und anschließend ansäuert.

Die als Ausgangsverbindungen benötigten ß-Aminoacrylsäureester sind bekannt oder lassen sich nach im Prinzip bekannten Verfahren herstellen (vgl. DT-OS 21 37 649 und DT-OS 22 53 027).

Die weiterhin als Reaktionskomponenten benötigten Verbindungen der Formel (V) sowie das Chlorcarbonylsulfenylchlorid der Formel (III) sind ebenfalls bekannt oder nach im Prinzip bekannten Verfahren herstellbar (vgl. Synthesis 1970, 567).

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn durch eine Dämpfung des Graswachstums kann z.B. die Häufigkeit der Grasschnitte in Ziergärten, Park- und Sportanlagen oder an Straßenrändern reduziert werden. Von Bedeutung ist auch die Hemmung des Wuchses von krautigen und holzigen Pflanzen an Straßenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so daß ein Mehrertrag bezogen auf die Bodenfläche erzielt werden kann.

Ein weiterer Mechanismus der Ertragssteigerung mit Wuchshemmern beruht darauf, daß die Nährstoffe in stärkerem Maße der Blüten- und Fruchtbildung zugute kommen, während das vegetative Wachstum eingeschränkt wird.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, so daß z.B. mehr oder größere Früchte zur Ausbildung kommen.

Ertragssteigerungen können in manchen Fällen auch durch einen Eingriff in den pflanzlichen Stoffwechsel erreicht werden, ohne daß sich Änderungen des vegetativen Wachstums bemerkbar machen. Wachstumsregulatoren können ferner eine Veränderung der Zusammensetzung der Pflanzen bewirken, um so eine bessere Qualität der Ernteprodukte herbeizuführen. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluß von Wachstumsregulatoren kann es zur Ausbildung parthenokarper Früchte kommen. Ferner kann das Geschlecht der Blüten beeinflußt werden.

Mit Wachstumsregulatoren läßt sich auch die Produktion oder der Abfluß von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen,

z.B. um bei Tabakpflanzen nach der Dekapitierung die Ausbildung von Seitentrieben zu verhindern und damit das Blattwachstum zu fördern.

Unter dem Einfluß von Wachstumsregulatoren kann der Blattbestand von Pflanzen so gesteuert werden, daß ein Entblättern der Pflanzen zu einem gewünschten Zeitpunkt erreicht wird. Eine derartige Entlaubung ist von Interesse, um eine mechanische Beerntung, z.B. bei Wein oder Baumwolle, zu erleichtern oder um die Transpiration zu einem Zeitpunkt herabzusetzen, an dem die Pflanze verpflanzt werden soll.

Durch Einsatz von Wachstumsregulatoren läßt sich der vorzeitige Fruchtfall verhindern. Es ist jedoch auch möglich, den Fruchtfall, - zum Beispiel bei Obst -, im Sinne einer chemischen Ausdünnung bis zu einem bestimmten Ausmaß zu fördern. Wachstumsregulatoren können auch dazu dienen, um bei Kulturpflanzen zum Zeitpunkt der Ernte die zum Ablösen der Früchte erforderliche Kraft zu vermindern, so daß eine mechanische Beerntung der Pflanzen ermöglicht beziehungsweise eine manuelle Beerntung erleichtert wird.

Mit Wachstumsregulatoren läßt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden die Voraussetzungen dafür geschaffen, daß z.B. bei Tabak, Tomaten oder Kaffee, eine vollständige mechanische oder manuelle Beerntung in nur einem Arbeitsgang vorgenommen werden kann.

Durch Anwendung von Wachstumsregulatoren kann auch die Samen- oder Knospenruhe der Pflanzen, also die endogene Jahresrhythmik, beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, daß der Austrieb von Knospen oder die Keimung von Samen verzögert wird, z.B. um in frostgefährdeten Gebieten eine Schädigung durch Spätfröste zu vermeiden.

Wachstumsregulatoren können auch eine Halophilie bei Kulturpflanzen erzeugen. Damit werden die Voraussetzungen dafür geschaffen, daß eine Kultivierung von Pflanzen auf salzhaltigen Böden durchgeführt werden kann.

Mit Wachstumsregulatoren kann auch eine Frost- und Trockenresistenz bei Pflanzen induziert werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide; Akarizide und Herbizide, sowie in Mischung mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Begasen usw. Es ist ferner möglich, die Wirkstoffe nach dem Ultra-Low-Volume-Verfahren auszubringen, Pflanzen oder Pflanzenteile mit der Wirkstoffzubereitung oder dem Wirkstoff selbst zu bestreichen oder die Wirkstoffzubereitung oder den Wirkstoff selbst in den Boden zu injizieren. Es kann auch das Saatgut der Pflanze behandelt werden.

Die Wirkstoffkonzentrationen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die erfindungsgemäß verwendbaren Stoffe der Formel (I) besitzen nicht nur pflanzenwachstumsregulierende Eigenschaften,

sondern zeichnen sich darüberhinaus auch durch fungizide Wirksamkeit aus.

In den nachfolgenden Beispielen wird die Aktivität der erfindungsgemäßen Stoffe als Wachstumsregulatoren dargestellt, ohne damit die Möglichkeit weiterer Anwendungen als Wachstumsregulatoren auszuschließen.

Beispiel A

Stimulation der Äthylenbiosynthese

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Aus Sojabohnenblättern werden Blattstücke gleicher Größe gestanzt. In Petrischalen, die mit 10 ml der Wirkstoffzubereitungen bzw. entsprechender Kontrollösungen ohne Wirkstoffe gefüllt sind, wird jeweils eine konstante Zahl Blattstücke 1 Stunde lang inkubiert. Anschließend werden die Blattstücke in luftdicht abgeschlossene Gefäße gegeben. Ein Teil der Wirkstoffzubereitungen wird ebenfalls in diese Gefäße gegeben. Nach 24 Stunden wird das Äthylen, das sich in den Gefäßen angesammelt hat, mit üblichen Nachweismethoden bestimmt. Die Äthylenentwicklung der mit Wirkstoffzubereitungen behandelten Blattstücke wird mit der Äthylenentwicklung der Kontrollen verglichen.

Das Pflanzenhormon Äthylen greift in zahlreiche Prozesse bei der Entwicklung der Pflanzen ein. Eine Erhöhung der Äthylenbiosynthese, wie sie mit den erfindungsgemäßen Substanzen erzielt werden kann, erlaubt es, diese Prozesse zu steuern. Als Beispiele, für die besonders ein kommerzielles Interesse besteht, seien hier genannt: Fruchtablösung, Reifebeschleunigung von Früchten und Blättern, Blühinduktion, Samenkeimung, Fruchtausdünnung, Stimmulation des Latexflusses z.B. bei Hevea und Wuchshemmung z.B. auch um das Lagern von Getreide zu verhindern.

Die Wirkstoffe der Formeln

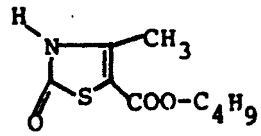

(12)

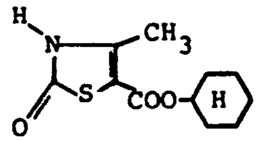

( 2)

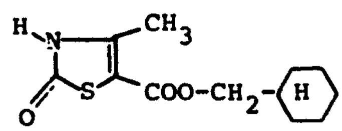

(15)

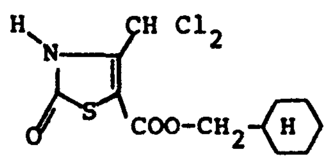

(34)

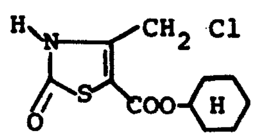

(37)

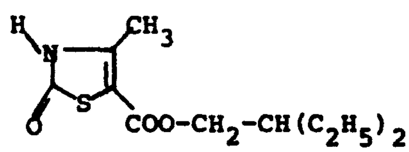

(38)

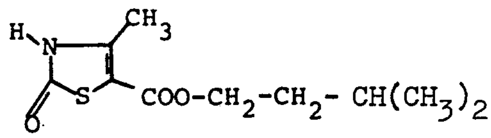

(33)

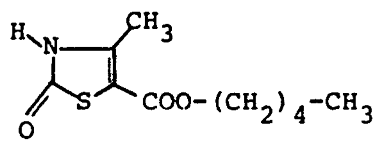

(39)

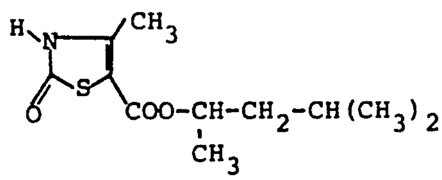

(40)

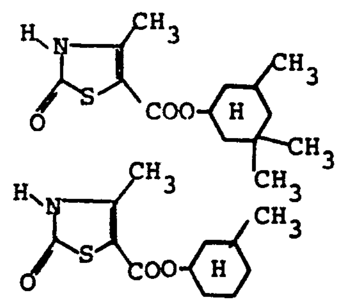

(42)

(43)

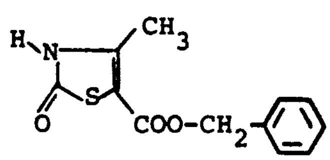

(11)

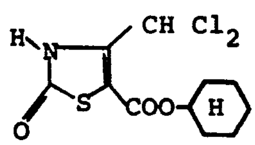

(35)

erhöhen die Äthylenbiosynthese im Vergleich zur Kontrolle.

Beispiel B

Reifebeschleunigung bei Tomaten

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Im Freiland werden Tomatenpflanzen in üblicher Weise angezogen bis die Hälfte der Früchte gefärbt ist. In diesem Stadium werden die Pflanzen bis zum Abtropfen mit den Wirkstoffzubereitungen besprüht. Nach 2 Wochen wird die Ausfärbung der Früchte im Vergleich zu der von unbehandelten Kontrollpflanzen bonitiert, und mit Kennziffern von 0 bis 3 bezeichnet, welche folgende Bedeutung haben:

0 = keine Reifebeschleunigung (wie unbehandelte Kontrolle)

1 = geringe Reifebeschleunigung

2 = mittlere Reifebeschleunigung

3 = starke Reifebeschleunigung

Wirkstoffe, Wirkstoffkonzentrationen und Resultate gehen aus der nachfolgenden Tabelle hervor.

**Tabelle**

Reifebeschleunigung bei Tomaten

| Wirkstoff | Wirkstoff-konzentration in ppm | Reifebe-schleunigung |
|---|---|---|
| - (Kontrolle) | - | 0 |
| 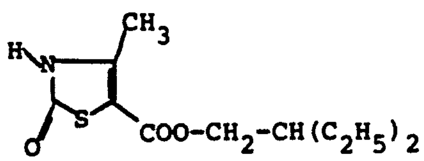 (38) | 2000 | 2 |

Beispiel C

Wuchshemmung bei Sojabohnen

Lösungsmittel: 30 Gewichtsteile Dimethylformamid
Emulgator: 1 Gewichtsteil Polyoxyäthylen- Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen bis zum Abtropfen mit den Wirkstoffzubereitungen besprüht. Nach 3 Wochen wird der Zuwachs gemessen und die Wuchshemmung in Prozent des Zuwachses der Kontrollpflanzen berechnet. 100 % bedeuten den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor.

Tabelle

Wuchshemmung bei Sojabohnen

| Wirkstoff | Wirkstoffkonzentration in % | Wuchshemmung in % |
|---|---|---|
| - (Kontrolle) | - | 0 |
| 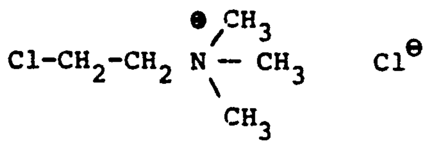 (bekannt) | 0,05 | 0 |
| 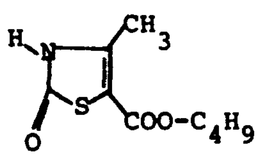 (12) | 0,05 | 35 |
| 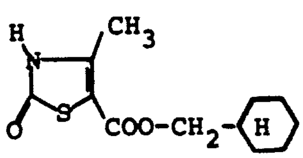 (15) | 0,05 | 35 |

| Wirkstoff | Wirkstoff-konzentration in % | Wuchshemmung in % |
|---|---|---|
| 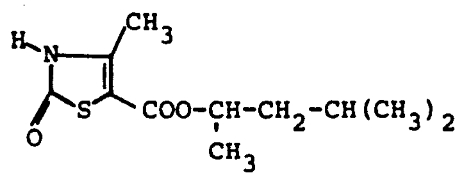 (40) | 0,05 | 25 |
| 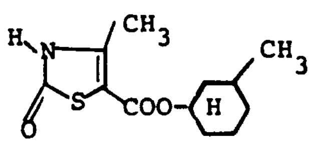 (43) | 0,05 | 25 |

**Beispiel D**

**Wuchshemmung bei Gehölzen** (Acer pseudoplatanus)

Lösungsmittel : 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Einjährige Sämlinge mit einer Wuchshöhe von 25 cm werden mit den Wirkstoffzubereitungen bis zum Abtropfen besprüht. Nach 6 Wochen Wachstum im Gewächshaus wird der Zuwachs gemessen und die Wuchshemmung in Prozent des Zuwachses der Kontrollpflanzen berechnet. 100 % bedeuten den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor.

Tabelle

Wuchshemmung bei Gehölzen (Acer pseudoplatanus)

| Wirkstoff | Wirkstoffkonzentration in % | Wuchshemmung in % |
|---|---|---|
| - (Kontrolle) | - | 0 |
| $HOOC-CH_2-CH_2-CO-NH-N(CH_3)_2$ (bekannt) | 1,0 | 20 |
| 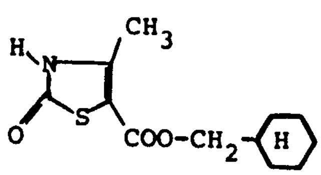 (15) | 0,4 | 45 |

Beispiel E

Wuchsförderung bei Weizen

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Weizenpflanzen werden im 2-Blatt-Stadium mit den Wirkstoffzubereitungen bis zum Abtropfen besprüht. Nach 3 Wochen Wachstum im Gewächshaus wird der Zuwachs gemessen und die Wuchsförderung in Prozent des Zuwachses der Kontrollpflanzen berechnet. Dabei bedeutet 0 % einen Zuwachs entsprechend dem der Kontrollpflanzen.

Wirkstoffe, Wirkstoffkonzentrationen und Resultate gehen aus der nachfolgenden Tabelle hervor.

Tabelle

Wuchsförderung bei Weizen

| Wirkstoff | | Wirkstoff-konzentration in % | Wuchsförderung in % |
|---|---|---|---|
| - (Kontrolle) | | - | 0 |
| 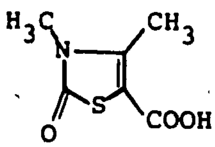 | (44) | 0,05 | 25 |

**Beispiel F**

**Wuchshemmung bei Baumwolle**

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Laubblattes angezogen. In diesem Stadium werden die Pflanzen bis zum Abtropfen mit den Wirkstoffzubereitungen besprüht. Nach 3 Wochen wird der Zuwachs der Pflanzen gemessen und die Wuchshemmung in Prozent des Zuwachses der Kontrollpflanzen berechnet. 100 % bedeuten den Stillstand des Wachstums und 0 % ein Wachstum entsprechend dem der Kontrollpflanzen.

Wirkstoffe, Wirkstoffkonzentrationen und Resultate gehen aus der nachfolgenden Tabelle hervor.

**Tabelle**

Wuchshemmung bei Baumwolle

| Wirkstoff | Wirkstoffkonzentration in % | Wuchshemmung in % |
|---|---|---|
| - (Kontrolle) | - | 0 |
| 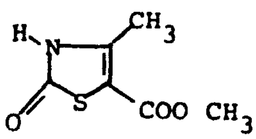 (13) | 0,05 | 45 |
| 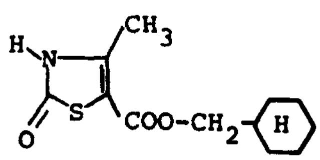 (15) | 0,05 | 95 |
| 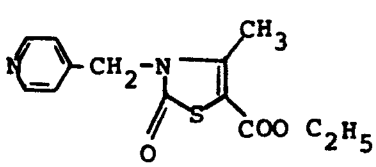 HCl (45) | 0,05 | 75 |

| Wirkstoff | Wirkstoff-konzentration in % | Wuchshemmung in % |
|---|---|---|
| 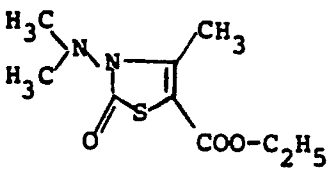 (36) | 0,05 | 35 |

Beispiel G

Wuchsförderung bei Baumwolle

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Laubblattes angezogen. In diesem Stadium werden die Pflanzen bis zum Abtropfen mit den Wirkstoffzubereitungen besprüht. Nach 3 Wochen wird der Zuwachs der Pflanzen gemessen und die Wuchsförderung in Prozent des Zuwachses der Kontrollpflanzen berechnet. Dabei bedeutet 0% einen Zuwachs entsprechend dem der Kontrollpflanzen.

Wirkstoffe, Wirkstoffkonzentration und Resultate gehen aus der nachfolgenden Tabelle hervor.

Tabelle

Wuchsförderung bei Baumwolle

| Wirkstoff | Wirkstoff-konzentration in % | Wuchsförderung in % |
|---|---|---|
| - (Kontrolle) | - | 0 |
| 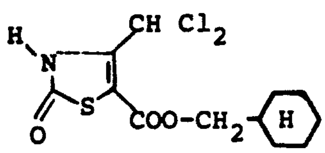 (34) | 0,05 | 25 |

Herstellungsbeispiele

Beispiel 1

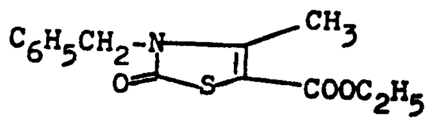

72 g (0,55 Mol) Chlorcarbonylsulfenylchlorid und 120 ml trockenes Chlorbenzol werden unter Eiskühlung bei 10° C bis 20°C tropfenweise mit 109,5 g (0,5 Mol) ß-Benzylaminocrotonsäureäthylester versetzt. Darauf wird etwa 1 Stunde auf 80°C bis 90°C erhitzt, wobei heftige Chlorwasserstoff-entwicklung eintritt. Man erhitzt zuletzt bis zur Be-endigung der Gasentwicklung zum Sieden, filtriert heiß und zieht das Lösungsmittel im Wasserstrahlvakuum ab. Das zurückbleibende Öl wird einer fraktionierten Hochvakuum-destillation unterworfen. Man erhält 91 g (66 % der Theorie) an 3-Benzyl-4-methyl-2-thiazolon-5-carbonsäureäthylester vom Siedepunkt 197 - 199°C / 0,5 Torr.

Beispiel 2

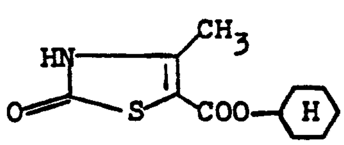

72 g (0,55 Mol) Chlorcarbonylsulfenylchlorid werden in 100 ml trockenem Chlorbenzol vorgelegt. Unter Eiskühlung und Rühren versetzt man tropfenweise mit 91,5 g (0,5 Mol) ß-Aminocrotonsäure-cyclohexylester. Man erhitzt vorsichtig

zum Sieden, wobei die Chlorwasserstoffentwicklung nicht zu heftig werden darf, und kocht dann rückfließend bis zur Beendigung der Gasentwicklung. Das Lösungsmittel wird im Vakuum abdestilliert und der Rückstand aus wenig Glykolmonoäthylätheracetat umkristallisiert. Es werden 72,5 g (60 % der Theorie) 4-Methyl-2-thiazolon-5-carbonsäure-cyclohexylester vom Schmelzpunkt 131°C erhalten.

In analoger Weise erhält man die folgenden Verbindungen der allgemeinen Formel

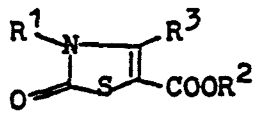

(I)

Tabelle 1

| Beispiel Nr. | $R_1$ | $R_2$ | $R_3$ | Fp. ($^{o}C$) Kp./Torr | Ausb. (%d.TH.) |
|---|---|---|---|---|---|
| 3 | $CH_3$ | $C_2H_5$ | $CH_3$ | 62 - 63 | 82 |
| 4 | H | $C_2H_5$ | $CH_3$ | 178 | 75 |
| 5 | H | $(CH_3)_2CH$ | $CH_3$ | 136 | 60 |
| 6 | H | $C_6H_5CH_2CH_2$ | $CH_3$ | 141 | 55 |
| 7 | $C_6H_5$ | $C_2H_5$ | $CH_3$ | 92 - 94 | 75 |
| 8 | $C_6H_5$ | $CH_3$ | $CH_3$ | 139 | 78 |
| 9 | H | $n\text{-}C_{12}H_{25}$ | $CH_3$ | 94 | 70 |
| 10 | 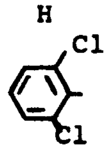 | $C_2H_5$ | $CH_3$ | 92 | 65 |
| 11 | H | $C_6H_5CH_2$ | $CH_3$ | 132 | 45 |
| 12 | H | $n\text{-}C_4H_9$ | $CH_3$ | 96 | 65 |
| 13 | H | $CH_3$ | $CH_3$ | 210 | 58 |
| 14 | H | $CH_3$ | $CCl_3$ | 128 | 42 |

**Tabelle 1** (Fortsetzung)

| Beispiel Nr. | $R_1$ | $R_2$ | $R_3$ | Fp. (°C) Kp./Torr | Ausbeute (% d.Th.) |
|---|---|---|---|---|---|
| 15 | H | $CH_2$— 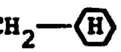 | $CH_3$ | 137 | 85 |
| 16 | H | $C_2H_5-O-(CH_2)_2$ | $CH_3$ | 89 | 60 |
| 17 | H | $(CH_3)_2CH-O-(CH_2)_2$ | $CH_3$ | 96 | 80 |
| 18 | H | $(CH_3)_2CH-CH_2$ | $CH_3$ | 122 | 76 |
| 19 | H | $CH_3(CH_2)_5$ | $CH_3$ | 94 | 84 |
| 20 | H | $NC-CH_2-CH_2$ | $CH_3$ | 143 | 30 |
| 21 | H | $CH_3-(CH_2)_9$ | $CH_3$ | 96 | 88 |
| 22 | H | $C_6H_5-O-(CH_2)_2$ | $CH_3$ | 155 | 85 |
| 23 | $CH_3$ | 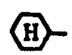 | $CH_3$ | 86 | 90 |
| 24 | $C_6H_5$ | 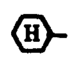 | $CH_3$ | 135 | 65 |

<u>**Tabelle 1**</u> **(Fortsetzung)**

| Beispiel Nr. | $R_1$ | $R_2$ | $R^3$ | Fp. (°C) Kp./Torr | Ausbeute (% d.Th.) |
|---|---|---|---|---|---|
| 25 | H | $CH_3(CH_2)_{17}$ | $CH_3$ | 105 | 80 |
| 26 | H | $C_2H_5$ | $CH_3(CH_2)_{16}$ | 42 | 75 |
| 27 | $CH_3$ | $CH_3$ | $(CH_3)_2CH-O-(CH_2)_2$ | 174/0,3 | |
| 28 | $CH_3-O-(CH_2)_2$ | $CH_3$ | $C_2H_5$ | 65 | |
| 29 | $C_6H_5$ | $CH_3$ | $(CH_3)_2CH-O-(CH_2)_2$ | 72 | |
| 30 | H | $CH_3$ | $n-C_4H_9-O-(CH_2)_2$ | 70 | |
| 31 | H | $CH_3$ | $n-C_3H_7-O-(CH_2)_2$ | 69 | |
| 32 | H | $CH_3$ | $CH_3-CH(OCH_3)-(CH_2)_2$ | 84 | |
| 33 | H | $(CH_2)_2-CH(CH_3)_2$ | $CH_3$ | 112-113 | |
| 34 | H | $CH_2$- 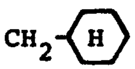 | $CHCl_2$ | 165-167 | |

**Tabelle 1 (Fortsetzung)**

| Beispiel Nr. | $R^1$ | $R^2$ | $R^3$ | Fp (°C) |
|---|---|---|---|---|
| 35 | H | 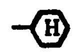 | $-CHCl_2$ | 187 |
| 36 | $-N(CH_3)_2$ | $C_2H_5$ | $CH_3$ | 57 |
| 37 | H | 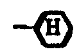 | $-CH_2Cl$ | 155 |
| 38 | H | $-CH_2-CH(C_2H_5)_2$ | $-CH_3$ | 106 |
| 39 | H | $-(CH_2)_4CH_3$ | $-CH_3$ | 99 |
| 40 | H | $-CH(CH_3)-CH_2-CH(CH_3)_2$ | $-CH_3$ | 89 |
| 41 | 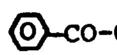-CO-$CH_2$ | $-(CH_2)_{11}-CH_3$ | $-CH_3$ | 83 |
| 42 | H | 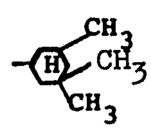 | $-CH_3$ | 134 |
| 43 | H | 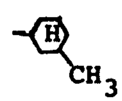 | $-CH_3$ | 97 |

Beispiel 44

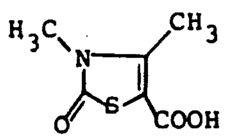

Eine Lösung von 201g (1 Mol) 3,4-Dimethyl-2-thiazolon-5-carbonsäureäthylester in 300 ml Äthanol wird mit 57 g Kaliumhydroxid gelöst in 180 ml Wasser versetzt. Man läßt 65 Stunden bei Raumtemperatur stehen, destilliert den Alkohol im Vakuum ab, löst den Rückstand im Wasser, filtriert und säuert unter Eiskühlung mit halbkonzentrierter Salzsäure an. Das Festprodukt wird abgesaugt, mit Wasser gewaschen und getrocknet.
Man erhält 162,5 g an 3,4-Dimethyl-2-thiazolon-5-carbonsäure vom Schmelzpunkt 214°C.

Beispiel 45

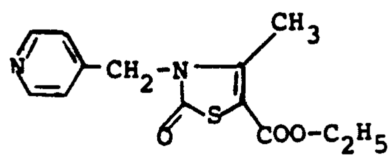

· HCl

Eine Lösung von 45 g Chlorcarbonylsulfenylchlorid in 100 ml trockenem Toluol wird unter Eiskühlung und Rühren tropfenweise mit 66 g (0,3 Mol) ß-(4-Pyridylmethylamino)-crotonsäureäthylester versetzt. Man erhitzt bis zur Beendigung der Gasentwicklung unter Rückfluß, destilliert das Lösungsmittel im Vakuum ab und verrührt den Rückstand mit kaltem Äthanol. Der Niederschlag wird abgesaugt und mit kaltem Alkohol gewaschen.
Man erhält 42,9 g an 3-(4-Pyridylmethyl)-4-methyl-2-thiazolon-5-carbonsäureester-hydrochlorid vom Schmelzpunkt 172 - 173°C.

Patentansprüche

1. Mittel zur Regulierung des Pflanzenwachstums, gekennzeichnet durch einen Gehalt an mindestens einem 2-Thiazolon-5-carbonsäure Derivat der Formel

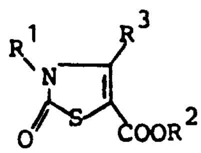

(I)

in welcher

$R^1$ für Wasserstoff, Alkyl, Alkoxyalkyl, Alkylmercaptoalkyl, durch heterocyclische Reste substituiertes Alkyl, durch Alkylcarbonyl substituiertes Alkyl, durch Arylcarbonyl, welches seinerseits im Arylteil durch Halogen, Alkyl, Halogenalkyl, Alkoxy, Alkylthio und/oder Nitro substituiert sein kann, substituiertes Alkyl, ferner für Alkenyl, Cycloalkyl, gegebenenfalls substituiertes Aryl, gegebenenfalls im Arylteil substituiertes Aralkyl oder für die Gruppierung

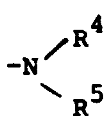

steht,

in welcher

$R^4$ für Wasserstoff, Alkyl oder Aryl steht,

$R^5$ für Alkyl, Alkylcarbonyl, Carbalkoxy, Alkylsulfonyl oder Arylsulfonyl steht und außerdem

$R^4$ und $R^5$ gemeinsam mit dem angrenzenden Stickstoffatom für einen 5 - bis 7 - gliedrigen heterocyclischen Ring stehen,

$R^2$ für Wasserstoff, gegebenenfalls durch Cycloalkyl, Alkoxy, Alkylmercapto, Arylthio oder Cyano substituiertes Alkyl gegebenenfalls durch Alkyl substituiertes Cycloalkyl oder für gegebenenfalls substituiertes Aralkyl steht und

$R^3$ für Wasserstoff, Alkyl, Alkoxyalkyl, Halogenalkyl, Cycloalkyl, gegebenenfalls substituiertes Aryl oder gegebenenfalls substituiertes Aralkyl steht.

2. Verfahren zur Regulierung des Pflanzenwachstums, dadurch gekennzeichnet, daß man 2-Thiazolon-5-carbonsäure-Derivate gemäß Anspruch 1 auf Pflanzen oder ihren Lebensraum einwirken läßt.

3. Verwendung von 2-Thiazolon-5-carbonsäure-Derivate gemäß Anspruch 1 zur Regulierung des Pflanzenwachstums.

4. Verfahren zur Herstellung von pflanzenwachstumsregulierenden Mitteln, dadurch gekennzeichnet, daß man 2-Thiazolon-5-carbonsäure-Derivate gemäß Anspruch 1 mit Streckmitteln und/oder oberflächenaktiven Mitteln vermischt.